# EUROPEAN PATENT SPECIFICATION

(11) **EP 2 229 973 B1**
(45) Date of publication and mention of the grant of the patent: **15.08.2012**
(21) Application number: 09015771.0
(22) Date of filing: 21.12.2009
(51) Int. Cl.: A61M 16/10, A61M 16/16

(54) **System for conditioning respiratory gases**
System zur Aufbereitung von Atemgas
Système pour le conditionnement de gaz respiratoires

(30) Priority: 17.03.2009 IT TO20090200
(43) Date of publication of application: 22.09.2010
(73) Proprietor: Covidien AG, 8212 Neuhausen am Rheinfall (CH)
(72) Inventor: Tralli, Stefano, 46022 Felonica (IT); Zucchi, Giuseppe, 41039 S. Possidonio (IT); Borali, Stefano, 41037 Mirandola (IT)
(74) Representative: Olsson, Carl H.S.

(56) References cited:
- EP-A- 1 994 952
- EP-A- 2 039 387
- WO-A-03/099367
- WO-A-2006/127257
- US-A- 2 584 450
- US-A- 5 482 031

## Description

### FIELD

The present invention relates to a system for conditioning respiratory gases.

The system and method are intended for use in Intensive Care to provide the right moisture/temperature level of gases inhaled by intubated patients under artificial ventilation.

The present invention may be used to particular advantage, though not exclusively, in Anaesthesiology and Intensive Care Unit (ICU), to which the following description refers purely by way of example.

### PRIOR ART

At present, the respiratory tracts of intubated patients under artificial ventilation in Intensive Care Units are heated and humidified using two main methods, depending on how long the patient is expected to be kept in Intensive Care.

A first passive conditioning system employing a heat and moisture exchanger (HME) is used when the patient is expected to remain in Intensive Care for roughly less than 72 hours.

As is known, an HME operates by retaining moisture and heat from the gases exhaled by the patient, and yielding most of the retained moisture and heat to the patient at the next inhalation stage.

Devices of this sort are certified to supply patients with an absolute moisture level of 28 to 33 mg/l, at a temperature ranging between 28 and 31°C, and to maintain correct respiration physiology for roughly 72 hours' treatment.

Operation of these devices normally remains stable for 24 hours, after which, the patient may experience difficulty in breathing (increase in work of breathing, WOB) caused by an increase in flow resistance, thus justifying replacement of the device every 24 hours.

A second respiratory gas conditioning system is based on active humidification.

The best currently marketed device provides for heating and humidifying gas supply to the patient to an absolute moisture level of 40 mg/l or more, and a temperature ranging between 35 and 39°C, and calls for very little maintenance, by temperature-regulating the expiration conduit to eliminate condensation.

An intermediate active device, operating in combination with an HME, however, provides for increasing heat and moisture supply to the patient by compensating the inhaled gas with a few mg of water vapour, thus enabling longer-term operation of the device (over 72 hours).

The HME system has the following advantages:
- less maintenance than an active device;
- adequate maintenance of correct respiration physiology for 72 hours;
- is easy to use;
The HME system may cause:
- "poor" humidification in most cases;
- augmentation of dead space within the respiratory circuit;
- possible augmentation in flow resistance, due to potential clogging (build-up of condensation) of the heat-exchange element.

The active-humidifier system has the following advantages:
- higher moisture supply as compared with a passive device;
- longer-term operation as compared with a passive device.
   The active-humidifier system may cause:

- possible over-humidification, caused by incorrect setting of the humidifier;
- high cost of the disposable-cartridge, of the circuit and of the sterile water;
- more frequent monitoring required than with passive devices;
- the moisture-sensitive flow sensors of the ventilator may have to be changed more frequently than usual, due to build-up of condensation on the expiration side, thus increasing operating cost;
- high consumption of sterile water.

A moisture-enrich HME device has the following advantages:
- higher moisture supply as compared with a passive device;
- consumes less sterile water than an active device.
   A moisture-enrich HME device may cause:

- additional bulk and weight of the HME, which are undesirable close to the patient.
   Various systems of the above types are described in WO2006/12725 (DHUPER et al.).

One embodiment in the above document, described with reference to Figures 4-6, employs an HME remote from the patient and combined with a number of temperature-regulated conduits.

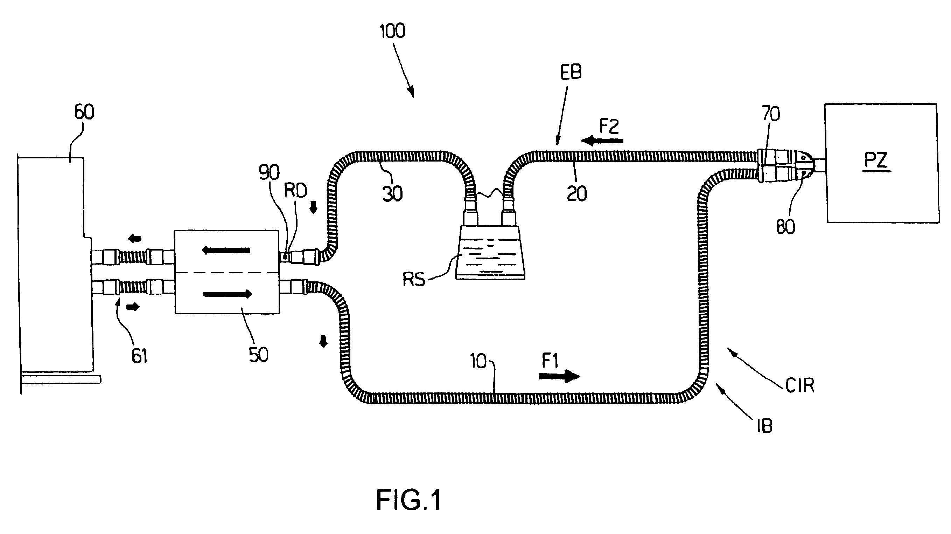

Another embodiment, shown in Figures 1-3, employs a device for injecting drugs into the device. Alternatively, an atomizer may be used.

Though successful, the systems described in WO2006/127257 have proved unreliable as regards precise regulation of the moisture level of the gas inhaled by the patient.

US 5,482,031 discloses an arrangement for connecting a patient to a respirator comprising a moisture-heat-exchanger and an active air humidifier being arranged between two inspiring hoses.

EP 2 039 387 A1 discloses a respiratory gas conditioning system with an inhale branch and an exhale branch, both being connected to a heat and moisture exchanger, wherein the exhale branch comprises a water reservoir to moisture-enrich the gas exhaled by the patient.

### SUMMARY

It is therefore an object of the present invention to ensure correct moisture supply to the patient. As is known, in this field, the basic parameters of the gas are moisture (i.e. the amount of water vapour per unit volume of gas) and temperature.

The main characteristic of the respiratory gas conditioning system according to the invention is defined by claim 1.

### DETAILED DESCRIPTION

Non-limiting embodiments of the present invention will be described by way of example with reference to the attached drawings.

As shown in the Figure 1, the system 100 according to the present invention comprises:
- three temperature-regulated conduits 10, 20, 30, one for an inhale branch IB, and two for an exhale branch EB;
- a water reservoir RS containing water possibly heated by an electric resistor (not shown), and which has top-up access, is characterized by containing a small amount of water, and is located along exhale branch EB;
- a heat and moisture exchanger (HME) 50, which is characterized by strict separation of the inhale flow F1 and exhale flow F2, is located close to a ventilator 60, and provides for separating the inhale/exhale flows while still ensuring correct heat and moisture exchange between the two, and with no increase in dead space in the circuit;
- a Y piece connector 70, which is located close to a patient PZ, connects the patient PZ to inhale branch IB and exhale branch EB, and has a socket for a temperature sensor 80 on inhale branch IB;
- a straight connector RD, with a socket for a temperature sensor 90, to connect HME 50 to exhale branch EB; and
- a thermostat (not shown) for the three temperature-regulated conduits 10, 20, 30. The term "thermostat" is intended herein to mean an electronic central control unit (not shown) connected electrically to temperature-regulated conduits 10, 20, 30 and temperature sensors 80, 90 to regulate the temperature of the gas flow to/from the patient PZ.

System 100 operates as follows:

Gas is exhaled by the patient PZ at roughly 32°C, and, as it flows along temperature-regulated exhale branch EB, is heated to a higher temperature, so as to be further enriched with moisture as it flows over the surface of the water inside reservoir RS.

The gas is then heated further, and is heated and humidified by the time it reaches HME 50 (close to ventilator 60) where the heat and moisture gradient assists release of heat and moisture to HME 50 itself.

Assuming a high-performance HME (Heat and Moisture Exchanger) 50 is used, enough heat and moisture is retained by the exchanger to supply ventilator 60 with relatively dry gas, and so eliminate the condensation well on the exhale line.

This therefore eliminates any problems with the moisture-sensitive flow sensor (not shown) forming part of ventilator 60.

At the next inhalation stage, the dry gas flowing through HME 50 from ventilator 60 is charged with heat and moisture and fed to the patient PZ along temperature-regulated inhale branch IB, which maintains the temperature of the gas to prevent the moisture in the gas from condensing.

In other words, the amount of heat and moisture in the gas supply to the patient PZ is controlled by adjusting the temperature of the gas flowing along inhale branch IB and exhale branch EB.

By determining the temperature of the gas supply to the patient PZ by means of temperature sensors 80, 90 installed along the circuit, the temperature of temperature-regulated conduits 10, 20, 30 can be controlled by a thermostat (not shown) as required by the patient PZ.

More specifically, heating temperature-regulated conduit 20 heats the gas exhaled by the patient PZ to gather more moisture from water reservoir RS; while heating the gas in temperature-regulated conduit 30 maintains the temperature and moisture level of the gas, and also produces a sufficient gradient between exhale branch EB and HME 50 to ensure effective transfer of heat and moisture to the exchange element (not shown) of HME 50. The exchange element, in turn, having a much higher heat and moisture content than the incoming gas from ventilator 60, heat and moisture are transferred to the inhale flow (F1) to the patient PZ, the conditions of such inhale flow (F1) are maintained along inhale branch IB by temperature-regulated conduit 10.

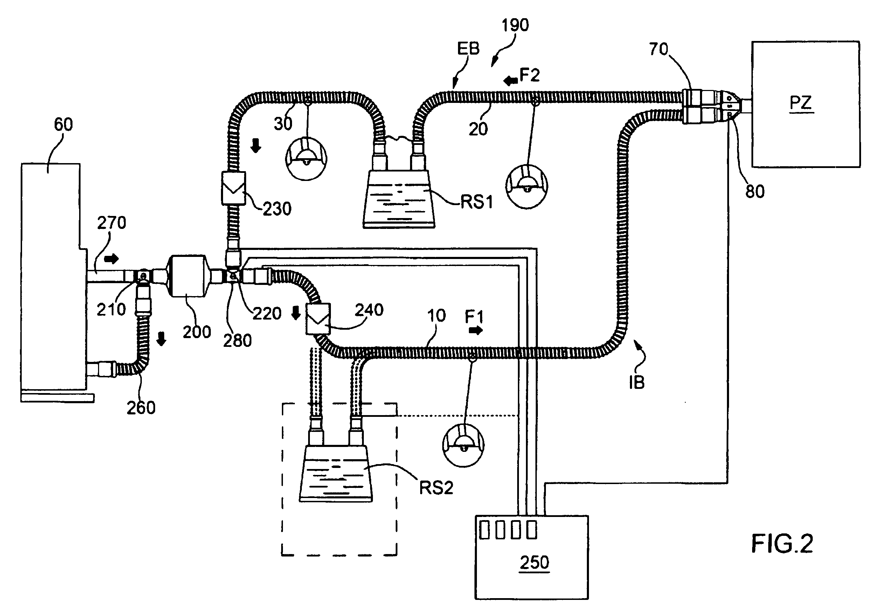

With reference to figure 2, the embodiment disclosed is similar with similar functions to the embodiment in figure 1 and the reference numerals in figure 1 correspond to the reference numerals in figure 2 to the extent they refer to the same components. The system 190 according to this embodiment comprises:
- at least three temperature-regulated conduits (heated wire tubes) 10, 20, 30, at least one for an inhale branch IB, 10 , and two for an exhale branch EB, 20, 30.
- at least a water reservoir RS1 containing water possibly heated by an electric resistor (not shown), and which is located along the exhale branch EB, 20, 30;
- a 'state-of-the art' heat and moisture exchanger (HME) 200 located close to a ventilator 60; this 'state-of-the art' HME uses the same channel for both inhalation and exhalation of air. It should be realized that an HME 200 having separate flow paths for inspiration and expiration could be used as well.
- a Y piece connector 70, which is located close to a patient P, connects the patient P to the inhale branch IB, 10 and the exhale branch EB, 20, 30 and has a socket for a temperature sensor 80 on the inhale branch IB. The heated wire tube 20 is connected to the Y-piece connector 70 as well as to the water reservoir RS1. The heated wire tube 30 is connected to the water reservoir RS1 as well as to a T-connector 220 via a one-way valve 230. The heated wire tube 10 is connected to the Y-piece connector 70 as well as the T-connector 220 via another one-way valve 240. The T-connector 220 is connected to the HME 200 which in turn is connected to another T-connector 210. The other T-connector 210 is connected to the ventilator 60 via an inlet tube 260 and an outlet tube 270. In an embodiment the inhale branch IB, 10 is connected between the Y-piece connector 70 and the T-connector 220 via another reservoir RS2 and a one-way valve 240. In this way the inspired air would be additionally humidified when flowing through the heated wire tube 10; through the water reservoir RS2 and towards the patient PZ. The one-way valve 240 located in the inhale branch (heated wire tube 10) is dimensioned so that air only can pass in one direction towards the Y-piece connector 70. Thus air cannot pass through the one-way valve 240 in the direction towards the HME 200. The one-way valve 230 is dimensioned so that air only can pass in one direction towards the T-connector 220 and the HME 200. Thus, air cannot pass through one-way valve 230 in the direction towards the water reservoir RS1. Consequently, expired air from the patient PZ will pass through the exhale branch EB, 20, 30; be heated up by the heated wires and humidified in the water reservoir RS1; continue through the one-way-valve 230 through the T-connector 220; through the HME; through the T-connector 210 and to the ventilator via the inlet tube 260; it should be noted that the inlet tube 260 could be a heated wire tube to avoid further condensation to reach the ventilator. While passing the HME 200, the humidity of the air will be absorbed and retained by the HME and dry air will eventually reach the ventilator. During the inspiration phase, the ventilator pushes the air through the outlet tube 270; through the T-connector 210; through the HME, where the humidity is released to the air. The humidified air passes through the other T-connector 220; through the one-way valve 240 and through the inhale branch IB, 10 where the humidified air is heated in order to avoid condensation of the air against the tube walls; eventually the air reaches the patient PZ via the Y-piece connector 70. In case the air needs to have a higher content of humidification a reservoir RS2 could be arranged in the inhale branch 10 between the one-way valve 240 and the Y-piece connector 70.
   It should be realized that in another embodiment (not disclosed), the water reservoir RS1 is absent and the only water reservoir RS2 used in the system is the one in the inhale branch IB. In this embodiment, the exhale tube EB is connected between the Y-connector 70 and the HME 200 without any water reservoir RS1; the inhale branch IB is connected via the water reservoir RS2 to the HME 200 and the patient P. Thus, inhaled gas from the ventilator 60 passes through the HME into the inhale branch IB and through the water reservoir RS2, where the gas is moisture-enriched; through the Y-connector 70 and to the patient. It has been shown that having the water reservoir RS2 in the inhale branch IB instead of the exhale branch EB could be very beneficial since the air is humidified just before reaching the patient PZ.

A heating regulation control unit 250 is connected to the different heated wire tubes 30, 10, and sensors, probes 80, 280 to regulate the temperature of the air flow in the circuit. The management of the quantity of the heat and moisture content of the gases provided to the patient PZ is in one embodiment handled by changing the temperature of the gasses passing inside the exhale and inhale branches. Thanks to the measurement of the temperature of the gases by means of the probes and sensors placed along the circuit 190, 100, it is possible to regulate the temperature by the thermo-regulator (heating regulation control unit) 250, depending upon the needs of the patient.

It should be realized that the breathing circuits according to the disclosed embodiments in figures 1 and 2, respectively, significantly decrease the condensation in the exhale hose and the inlet tube 260 (only disclosed in figure 2). From a practical point of view it is very convenient to have the HME on the ventilator side since it reduces weight and bulk near the patient. It should be realized that using an HME 200, 50, when humidity is actively delivered RS1, RS2 to a patient, only makes sense if the HME is located close to the ventilator (60); in case the HME 50, 200 is positioned close to the patient PZ, it would be clogged immediately.

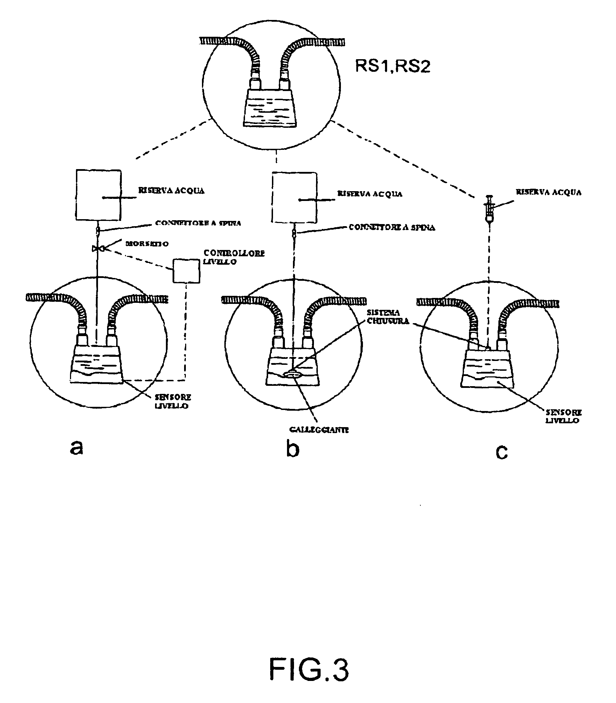

Figure 3 refers to regulating the water level in the water reservoir RS1, RS2. Figure 3a represents an automatic fill-up system with a level sensor inside the reservoir, said sensor being connected to a level controller. At a certain 'low' water level, the level sensor communicates with level controller activating a clamp connected to a water supply whereby the reservoir is filled up with water. In figure 3b, the reservoir has a floater that is movable up and down based on the height of the water in the reservoir. The floater is connected to a water supply via a feeding line and a spike connector. When the floater is in a lowest position representing a low water level, the spike connector in the feeding line is opened whereby water is provided to the reservoir. When the floater is in a highest position representing enough water in the reservoir, the spike connector is closed preventing water from reaching the reservoir. Figure 3c discloses a manual system having a level sensor indicating when it is time to fill up the reservoir. Normally the reservoir is in this embodiment filled up with a syringe.

The main advantages of the respiratory gas conditioning system according to the present invention are as follows:

- low energy consumption as compared with a conventional active humidifier; energy, in fact, is only used to heat the temperature-regulated conduits and possibly slightly heat the water reservoir;

- low water consumption as compared with a conventional active humidifier; the system, in fact, only supplies the amount of moisture needed to compensate moisture loss by the patient exhaling;

- very few routine checks, thus reducing system maintenance as compared with both passive and active devices;

- elimination of conventional system water traps; by virtue of the high performance of the HME, the exhale-side gas is dry enough to eliminate the condensation well; and calibrating moisture content to simply compensate consumption prevents the formation of surplus moisture, and so eliminates the need for a condensation well along the inhale branch;

- longer-term operation of the system as compared with a conventional HME;

- adequate heating and humidification of the patient-inhaled gases; the amount of moisture added by the enrich system, in fact, compensates the moisture loss of the HME, thus supplying the patient with the required moisture level;

- improved patient safety; the low power employed and the small amount of added moisture safeguard the patient against scalding and surplus moisture;

- complete separation of the inhale and exhale flows of the HME reduces 'dead-space' in the circuit and enables elimination of one-way valves from the circuit as is disclosed in figure 1;

- lighter weight of the circuit close to the patient; unlike other humidifiers, the HME is located close to the ventilator, as opposed to the patient; and eliminating the water traps, which fill up with water, further reduces the weight of the circuit weighing on the patient;

- the system is also ideal for use with new-born babies, being so flexible and effective to humidify and heat even small gas flows by simply increasing temperature regulation of the conduits.

## Claims

1. A respiratory gas conditioning system (190) comprising:
an inhale branch (IB) and an exhale branch (EB);
an HME (200) located close to a ventilator (60);
each of said inhale branch (IB) and said exhale branch (EB) being connected to the HME (200) and, by means of a connector (70), to a patient (P);
said inhale branch comprises an inhale tube (10) connected between the connector (70) and the HME (200);
said exhale branch (EB) comprises a first water reservoir (RS1), said first water reservoir (RS1) being arranged to moisture-enrich the gas flowing along said exhale branch (EB), **characterized in that**,
said inhale branch (IB) comprises a second water reservoir (RS2), said second water reservoir (RS2) being arranged to moisture-enrich the gas flowing along said inhale branch (IB).

2. A system (190), as claimed in Claim 1, **characterized in that**, a first exhale tube (20) connected between the connector (70) and the first water reservoir (RS1) and a second exhale tube (30) connected between the first water reservoir (RS1) and the HME (200).

3. A system (190), as claimed in any of the preceding Claims, **characterized in that** said tubes being temperature-regulated.

4. A system (190), as claimed in any of the Claims 2 or 3, **characterized in that** said first water reservoir (RS1) contains a given amount of water, which contacts with the exhaled gas flowing from the first exhale tube (20) to the second exhale tube (30).

5. A system (190), as claimed in any of the preceding Claims, **characterized in that** the water in the water reservoir (RS1,RS2) is heated by an electric resistor.

6. A system (190), as claimed any of the preceding Claims, **characterized in that** said exhale branch (EB,30) comprises a first one-way valve (230) allowing the gas to flow only in the direction towards the HME (200) away from the patient.

7. A system (190), as claimed in any of the preceding Claims, **characterized in that** said inhale branch (IB,10) comprises a second one-way valve (240) allowing the inspired gas to flow only in the direction away from the HME towards the patient (PZ).

8. A system (190), as claimed in any of the preceding Claims, **characterized in that** said exhale branch (EB,30) and said inhale branch (IB,10) are connected to the HME (200) via a first joint connector (220).

9. A system (190), as claimed in Claim 8, **characterized in that** said HME (200) is connected between said first joint connector (220) and said ventilator (60).

10. A system (190), as claimed in any of the preceding Claims, **characterized in that** said second water reservoir (RS2) is connected to said inhale tube (10) between the HME (200) and the connector (70).

11. A system (190), as claimed in any of the preceding Claims, **characterized in that** the HME (200) has a configuration separating the inhale gas flow and exhale gas flow.

12. A system (190), as claimed in any of the preceding Claims, **characterized in that** it comprises a heating controller (250) for monitoring the temperature in the system (10,20,30,80,280) and temperature-regulating the gas flow in the system maintaining adequate humidification.

## Patentansprüche

1. Atemgasaufbereitungssystem (190), enthaltend:
einen Einatmungszweig (IB) und einen Ausatmungszweig (EB);
einen HME (200), der nahe einem Beatmungsgerät (60) angeordnet ist;
wobei sowohl der Einatmungszweig (IB) als auch der Ausatmungszweig (EB) an den HME (200) und mittels eines Verbindungsstücks (70) an einen Patienten (P) angeschlossen sind;
wobei der Einatmungszweig einen Einatmungsschlauch (10) aufweist, der zwischen dem Verbindungsstück (70) und dem HME (200) angeschlossen ist;
wobei der Ausatmungszweig (EB) einen ersten Wasserbehälter (RS1) enthält, wobei der erste Wasserbehälter (RS1) zur Anreicherung des Gases, das entlang dem Ausatmungszweig (EB) strömt, mit Feuchtigkeit ausgebildet ist, **dadurch gekennzeichnet, dass**:
der Einatmungszweig (IB) einen zweiten Wasserbehälter (RS2) enthält, wobei der zweite Wasserbehälter (RS2) zur Anreicherung des Gases, das entlang dem Einatmungszweig (IB) strömt, mit Feuchtigkeit ausgebildet ist.

2. System (190) nach Anspruch 1, **dadurch gekennzeichnet, dass** ein erster Ausatmungsschlauch (20) zwischen dem Verbindungsstück (70) und dem ersten Wasserbehälter (RS1) angeschlossen ist und ein zweiter Ausatmungsschlauch (30) zwischen dem ersten Wasserbehälter (RS1) und dem HME (200) angeschlossen ist.

3. System (190) nach einem der vorangehenden Ansprüche, **dadurch gekennzeichnet, dass** die Schläuche temperaturgeregelt sind.

4. System (190) nach einem der Ansprüche 2 oder 3, **dadurch gekennzeichnet, dass** der erste Wasserbehälter (RS1) eine bestimmte Wassermenge enthält, die mit dem ausgeatmeten Gas, das vom ersten Ausatmungsschlauch (20) zum zweiten Ausatmungsschlauch (30) strömt, in Kontakt gelangt.

5. System (190) nach einem der vorangehenden Ansprüche, **dadurch gekennzeichnet, dass** das Wasser in dem Wasserbehälter (RS1, RS2) durch einen elektrischen Widerstand erwärmt wird.

6. System (190) nach einem der vorangehenden Ansprüche, **dadurch gekennzeichnet, dass** der Ausatmungszweig (EB, 30) ein erstes Einwegventil (230) enthält, das das Gas nur in die Richtung zum HME (200), weg vom Patienten, strömen lässt.

7. System (190) nach einem der vorangehenden Ansprüche, **dadurch gekennzeichnet, dass** der Einatmungszweig (IB, 10) ein zweites Einwegventil (240) enthält, das das eingeatmete Gas nur in die Richtung, weg vom HME, zum Patienten (PZ) strömen lässt.

8. System (190) nach einem der vorangehenden Ansprüche, **dadurch gekennzeichnet, dass** der Ausatmungszweig (EB, 30) und der Einatmungszweig (IB, 10) über ein erstes gemeinsames Verbindungsstück (220) an den HME (200) angeschlossen sind.

9. System (190) nach Anspruch 8, **dadurch gekennzeichnet, dass** der HME (200) zwischen dem ersten gemeinsamen Verbindungsstück (220) und dem Beatmungsgerät (60) angeschlossen ist.

10. System (190) nach einem der vorangehenden Ansprüche, **dadurch gekennzeichnet, dass** der zweite Wasserbehälter (RS2) zwischen dem HME (200) und dem Verbindungsstück (70) an den Einatmungsschlauch (10) angeschlossen ist.

11. System (190) nach einem der vorangehenden Ansprüche, **dadurch gekennzeichnet, dass** der HME (200) einen Aufbau hat, der den Einatmungsgasstrom und den Ausatmungsgasstrom trennt.

12. System (190) nach einem der vorangehenden Ansprüche, **dadurch gekennzeichnet, dass** es einen Heizungsregler (250) zur Überwachung der Temperatur in dem System (10, 20, 30, 80, 280) und zur Temperaturregulierung des Gasstromes in dem System zur Aufrechterhaltung einer angemessenen Befeuchtung enthält.

## Revendications

1. Système (190) de conditionnement de gaz respiratoires, comportant :
une branche (IB) d'inhalation et une branche (EB) d'exhalation ;
un HME (200) situé près d'un respirateur (60) ;
chacune desdites branches (IB) d'inhalation et (EB) d'exhalation étant reliée au HME (200) et, au moyen d'un raccord (70), à un patient (P) ;
ladite branche d'inhalation comportant un tube (10) d'inhalation branché entre le raccord (70) et le HME (200) ;
ladite branche (EB) d'exhalation comportant un premier réservoir (RS1) d'eau, ledit premier réservoir (RS1) d'eau étant configuré pour enrichir en humidité le gaz circulant le long de ladite branche (EB) d'exhalation,
**caractérisé en ce que**
ladite branche (IB) d'inhalation comporte un deuxième réservoir (RS2) d'eau, ledit deuxième réservoir (RS2) d'eau étant configuré pour enrichir en humidité le gaz circulant le long de ladite branche (IB) d'inhalation.

2. Système (190) selon la revendication 1. **caractérisé par** un premier tube (20) d'exhalation branché entre le raccord (70) et le premier réservoir (RS1) d'eau et un deuxième tube (30) d'exhalation branché entre le premier réservoir (RS1) d'eau et le HME (200).

3. Système (190) selon l'une quelconque des revendications précédentes, **caractérisé en ce que** lesdits tubes sont régulés en température.

4. Système (190) selon l'une quelconque des revendications 2 ou 3, **caractérisé en ce que** ledit premier réservoir (RS1) d'eau contient une quantité donnée d'eau, qui entre en contact avec le gaz exhalé circulant du premier tube (20) d'exhalation au deuxième tube (30) d'exhalation.

5. Système (190) selon l'une quelconque des revendications précédentes, **caractérisé en ce que** l'eau se trouvant dans le réservoir (RS1, RS2) d'eau est chauffée par une résistance électrique.

6. Système (190) selon l'une quelconque des revendications précédentes, **caractérisé en ce que** ladite branche (EB, 30) d'exhalation comporte un premier clapet anti-retour (230) permettant au gaz de circuler uniquement en direction du HME (200) en s'éloignant du patient.

7. Système (190) selon l'une quelconque des revendications précédentes, **caractérisé en ce que** ladite branche d'inhalation (IB, 10) comporte un deuxième clapet anti-retour (240) permettant au gaz inspiré de circuler uniquement dans le sens s'éloignant du HME vers le patient (PZ).

8. Système (190) selon l'une quelconque des revendications précédentes, **caractérisé en ce que** ladite branche (EB, 30) d'exhalation et ladite branche d'inhalation (IB, 10) sont reliées au HME (200) via un premier raccord commun (220).

9. Système (190) selon la revendication 8, **caractérisé en ce que** ledit HME (200) est branché entre ledit premier raccord commun (220) et ledit respirateur (60).

10. Système (190) selon l'une quelconque des revendications précédentes, **caractérisé en ce que** ledit deuxième réservoir (RS2) d'eau est relié audit tube (10) d'inhalation entre le HME (200) et le raccord (70).

11. Système (190) selon l'une quelconque des revendications précédentes, **caractérisé en ce que** le HME (200) présente une configuration séparant l'écoulement de gaz d'inhalation et l'écoulement de gaz d'exhalation.

12. Système (190) selon l'une quelconque des revendications précédentes, **caractérisé en ce qu'**il comporte un régulateur (250) de chauffage destiné à surveiller la température dans le système (10, 20, 30, 80, 280) et à réguler en température l'écoulement de gaz dans le système en maintenant une humidification adéquate.
